# EUROPÄISCHE PATENTSCHRIFT

(11) **EP 3 600 464 B1**
(45) Veröffentlichungstag und Bekanntmachung des Hinweises auf die Patenterteilung: **04.05.2022**
(21) Anmeldenummer: 18712926.7
(22) Anmeldetag: 29.03.2018
(51) Int. Cl.: A61L 27/02, A61L 27/12, A61L 27/18, A61L 27/42, A61L 27/46, A61L 27/52, A61L 27/58

(54) **TRÄGERZUSAMMENSETZUNG FÜR KNOCHENERSATZMATERIALIEN**
CARRIER COMPOSITION FOR BONE SUBSTITUTE MATERIALS
COMPOSITION DE SUPPORT POUR MATÉRIAU DE SUBSTITUTION OSSEUSE

(30) Priorität: 29.03.2017 EP 17163687
(43) Veröffentlichungstag der Anmeldung: 05.02.2020
(73) Patentinhaber: ARTOSS GmbH, 18069 Rostock (DE)
(72) Erfinder: GERBER, Thomas, 18059 Papendorf (DE)
(74) Vertreter: Uexküll & Stolberg
(86) Internationale Anmeldenummer: PCT/EP2018/058132
(87) Internationale Veröffentlichungsnummer: WO 2018/178266

(56) Entgegenhaltungen:
- WO-A1-01/60409
- WO-A1-2011/137231
- WO-A1-2014/099967
- WO-A1-2014/157985
- WO-A2-2007/025698
- DATABASE WPI Week 201623 Thomson Scientific, London, GB; AN 2016-166488 XP002773868, -& WO 2016/037249 A1 (UNICAMP UNIV ESTADUAL CAMPINAS) 17. März 2016 (2016-03-17)
- ALVARO MUNOZ ET AL: "Localized inhibition of P2X7R at the spinal cord injury site improves neurogenic bladder dysfunction by decreasing urothelial P2X3R expression in rats", LIFE SCIENCES., Bd. 171, 1. Februar 2017 (2017-02-01), Seiten 60-67, XP055406456, GB ISSN: 0024-3205, DOI: 10.1016/j.lfs.2016.12.017
- KIENAST B ET AL.: "Nanostrukturiertes synthetisches Knochenmaterial zur Behandlung von Knochendefekten", TRAUMA UND BERUFSKRANKHEIT, SPRINGER-VERLAG, BERLIN/HEIDELBERG, Bd. 18, Nr. 4, 21. Oktober 2016 (2016-10-21), Seiten 308-318, XP036115702, ISSN: 1436-6274, DOI: 10.1007/S10039-016-0209-7 [gefunden am 2016-10-21]
- Anonymous: "Summary for ARTG Entry: 292263 Global Orthopaedic Technology Pty Ltd -NanoBone SBX Putty - Bone matrix implant, synthetic", The Therapeutic Goods Administration (TGA) - Australian Government Department of Health , 31. Juli 2017 (2017-07-31), XP055406921, Gefunden im Internet: URL:https://www.ebs.tga.gov.au/servlet/xml millr6?dbid=ebs/PublicHTML/pdfStore.nsf&do cid=292263&agid=(PrintDetailsPublic)&actio nid=1 [gefunden am 2017-09-14]
- Camila P. Silveira ET AL: "Doxorubicin-Functionalized Silica Nanoparticles Incorporated into a Thermoreversible Hydrogel and Intraperitoneally Administered Result in High Prostate Antitumor Activity and Reduced Cardiotoxicity of Doxorubicin", ACS Biomaterials Science & Engineering, vol. 2, no. 7, 11 July 2016 (2016-07-11), pages 1190-1199, XP055406459, US ISSN: 2373-9878, DOI: 10.1021/acsbiomaterials.6b00241
- C. P. Silveira ET AL: "Hybrid biomaterial based on porous silica nanoparticles and Pluronic F-127 for sustained release of sildenafil: in vivo study on prostate cancer", RSC Adv., vol. 5, no. 99, 1 January 2015 (2015-01-01), pages 81348-81355, XP055406712, DOI: 10.1039/C5RA15006J

## Beschreibung

### GEBIET DER ERFINDUNG

Die vorliegende Erfindung betrifft eine Trägerzusammensetzung für partikuläre und granuläre Knochenersatzmaterialien, bei der es sich um ein Hydrogel handelt, das ein Gemisch aus Ethylenoxid(EO)-Propylenoxid(PO)-Blockcopolymeren umfasst, sowie darin eingebettete Silica-Nanopartikel mit einer Größe zwischen etwa 0,5 nm und etwa 10 nm. Die vorliegende Erfindung betrifft ferner ein Knochenersatzmaterial, das neben der neuartigen Trägerzusammensetzung osteokonduktive und/oder osteoinduktive Partikel oder Granulate enthält. Ein Verfahren zur Herstellung der neuartigen Trägerzusammensetzung wird bereitgestellt. Ein Verfahren zur Herstellung des neuartigen Knochenersatzmaterials wird ebenfalls im Rahmen der Erfindung bereitgestellt.

### HINTERGRUND DER ERFINDUNG

Knochenersatzmaterialien haben eine Funktionalität, die durch ihre Struktur und Zusammensetzung bestimmt wird. Knochenersatzmaterialien entfalten ihre Wirkung durch Wechselwirkung der Materialoberfläche mit Proteinen, z.B. solchen, die den Knochenstoffwechsel steuern, und Oberflächenstrukturen, die eine Zelladhäsion fördern. Im Stand der Technik bekannte Knochenersatzmaterialien sind in der Regel Keramiken oder Biogläser, die meist in Granulatform verwendet werden. Vor der Anwendung werden diese Granulate mit dem Blut des Patienten gemischt, sodass die Oberfläche des Granulats mit autologen Proteinen belegt wird. Durch die Koagulation des Blutes entsteht eine pastöse Masse, die z.B. in Knochendefekte eingebracht werden kann.

Allerdings ist diese Herstellung dieser Knochenersatzmaterialien mit erheblichen Nachteilen verbunden. So erschweren die Notwendigkeit des Mischens des Knochenersatzmaterials mit Blut und das Warten auf die Koagulation regelmäßig den Ablauf einer Operation. Aus diesem Grund wurde im Stand der Technik versucht, ein Trägermaterial für Knochenersatzmaterialien zu entwickeln, dass das Vermischen mit Blut überflüssig macht. Hierbei ist es wichtig, die rheologischen Eigenschaften an die konkreten Anwendungen anpassen zu können. Einerseits sollte ein Trägermaterial für Knochenersatzgranulate möglichst form- und hydrostabil sein und auch nach Einbringen in einen stark blutenden Defekt hinreichende Klebewirkung entfalten. Andererseits sollte das Trägermaterial in einer Darreichungsform vorliegen, die eine Verabreichung mittels einer Kanüle ermöglicht.

WO 2004/071452 A2 beschreibt Poloxamere, wie z.B. Poloxamer 407, für die Anwendung im medizinischen und chirurgischen Bereich. WO 2012/117260 A2 offenbart ein synthetisches Knochenersatzmaterial, bei dem keramische Partikel in einem Hydrogel-Träger eingebettet sind. Bei dem Hydrogel handelt es sich vorzugsweise um ein Hydrogel auf Basis von Poloxamer 407. Auch WO 2014/099967 A2 beschreibt ein Knochenersatzmaterial, das keramische Bestandteile in einem auf Poloxamer 407 basierenden Hydrogel enthält. US 2016/0051725 (WO2014157985)offenbart ein Hydrogel auf Poloxamer-Basis, das Calciumphosphat-Partikel enthält, und beschreibt, wie die viskoelastischen und rheologischen Eigenschaften der Hydrogele durch Zusätze verbessert werden können. US 2013/0045920 (WO2011137231) beschreibt ein Knochenersatzmaterial, das ein keramisches Material, ein Poloxamer 407-Hydrogel sowie Polysaccharidzusätze umfasst. WO 2014/095915 A2 beschreibt ein thermoreversibles Hydrogel auf Poloxamer-Basis, das einen vielseitigen Einsatz auf medizinischem Gebiet ermöglichen soll. Die Gelbildung erfolgt bei diesen Hydrogelen über die temperaturabhängige Bildung von Mizellen durch die Blockpolymere. Die im Stand der Technik beschriebenen Hydrogele weisen jedoch den Nachteil auf, dass ihre Viskosität zu niedrig ist, um eine ausreichend hohe Formbarkeit und Klebrigkeit des Hydrogels zu gewährleisten.

Um eine reibungsfreie Anwendung der Knochenersatzmaterialien zu gewährleisten, wäre ein gebrauchsfertiges Trägermaterial erforderlich, das sich gut formen lässt und ausreichend klebrig ist, um in einem Defekt fixiert zu werden. Ferner sollte das Material hydrostabil sein, d.h. es sollte auch bei stark blutenden Wunden nicht weggespült werden. Im Idealfall sollte es direkt aus einem entsprechenden Applikator in den Defekt eingebracht werden können.

Der vorliegenden Anmeldung liegt somit die Aufgabe zu Grunde, ein Trägermaterial zu entwickeln, das den obigen Anforderungen entspricht.

### BESCHREIBUNG DER ERFINDUNG

Die vorliegende Erfindung stellt eine neuartige Zusammensetzung bereit, die sich als Träger für partikuläre und granuläre Knochenersatzmaterialien eignet. Bei der Trägerzusammensetzung handelt es sich um ein Hydrogel, das folgendes umfasst:
(a) ein Ethylenoxid(EO)-Propylenoxid(PO)-Blockcopolymer oder ein Gemisch aus Ethylenoxid(EO)-Propylenoxid(PO)-Blockcopolymeren; und
(b) Silica-Nanopartikel mit einer Größe zwischen etwa 0,5 nm und etwa 10 nm.

In den erfindungsgemäßen Hydrogelen erfolgt die Gelbildung über eine Vernetzung der Ethylenoxid(EO)-Propylenoxid(PO)-Blockcopolymere mit den Silica-Nanopartikeln. Durch gezielte Veränderung der Hydrogel-Bestandteile können die rheologischen Eigenschaften modifiziert und an unterschiedliche Anwendungen angepasst werden. Anders als bei üblichen Hydrogelen auf Basis von Blockcopolymeren erfolgt die Gelbildung bei den erfindungsgemäßen Hydrogelen nicht durch Mizellen-Bildung, sondern über direkte Wechselwirkungen zwischen den Silica-Nanopartikeln und den Blockcopolymeren. Die Hydrogele der vorliegenden Erfindung umfassen daher keinen oder lediglich einen geringen Anteil von Blockcopolymeren, die in Form von Mizellen vorliegen. Vorzugsweise liegt der Anteil von Blockcopolymeren, die in Form von Mizellen vorliegen, unterhalb von 2%, und stärker bevorzugt unterhalb von 1% Prozent. In einer besonders bevorzugten Ausführungsform weisen die Hydrogele der vorliegenden Erfindung keine Mizellen auf. Da die erfindungsgemäßen Hydrogele nicht auf einer Bildung von Mizellen beruhen, sind sie nicht thermosensitiv, d.h. der Sol-Gel-Übergang hängt bei diesen Hydrogelen nicht von der Temperatur ab. Die erfindungsgemäßen Hydrogele sind thermostabil und werden auch bei niedrigen Temperaturen nicht flüssig.

Es wurde im Rahmen der Erfindung festgestellt, dass die neue Trägerzusammensetzung die biologischen Prozesse der Knochenheilung nicht negativ beeinflusst. Es wurde keine negative Wechselwirkung der Trägerzusammensetzung mit der Oberfläche des Knochenersatzmaterials nachgewiesen, z.B. indem eine Belegung dieser Oberfläche mit autologen Proteinen verhindert oder Nanoporen verstopft werden. Darüber hinaus weist die neue Trägerzusammensetzung viskoelastische und rheologische Eigenschaften auf, die eine schnelle Resorption gewährleisten. Die neue Trägerzusammensetzung macht daher eine Mischung der bekannten Granulate mit Blut überflüssig. Vielmehr werden die Knochenersatzmaterialien in Granulatform direkt mit der neuen Trägerzusammensetzung gemischt und in den Defekt eingebracht. Dies führt zu einer wesentlichen Vereinfachung in der klinischen Praxis.

Bei der Trägerzusammensetzung handelt es sich um ein Hydrogel auf Basis von einem oder mehreren Blockcopolymere aus Ethylenoxid und Propylenoxid. Vorzugsweise handelt es sich bei den Blockcopolymeren um Poloxamere. Poloxamere sind schaumarme nicht-ionische Tenside, die beim Dispergieren und Emulgieren in der chemisch-technischen Industrie verbreitet Anwendung finden. Der Polyethylenoxidteil des Polymers ist wasserlöslich, der Polypropylenoxidteil jedoch nicht, so dass sich die amphiphilen Eigenschaften ergeben. Je nach Ethoxylierungsgrad sind sie flüssig (L), pastös (P), fest (F) oder pulverförmig. Poloxamere weisen eine gute Biokompatibilität auf, sind unter physiologischen Bedingungen nicht metabolisierbar, kaum toxisch oder korrosiv, und leicht aus dem Körper eliminiert.

Poloxamere wurden in den 1950er Jahren von BASF entwickelt und werden seither unter dem Markennamen Pluronic^{®} vertrieben. Poloxamere sind in wässrigen Mehrstoffgemischen aufgrund ihrer amphiphilen Struktur dazu in der Lage, sogenannte lyotrope Assoziationskolloide zu bilden. Hierbei tritt ein sogenanntes Thermogelierverhalten auf. Das heißt, Poloxamer-Lösungen können in Abhängigkeit von der Temperatur ihre kolloidale Struktur verändern und dabei reversible Gelstrukturen ausbilden. Bringt man Poloxamere in Kontakt mit Wasser, so findet zunächst eine Hydratation unter Ausbildung von Wasserstoffbrückenbindungen statt. Bei Temperaturerhöhung kommt es zu einer Verminderung der Bindungskräfte dieser Wasserstoffbrückenbindungen und somit zu einer Dehydratation, wobei vorwiegend die hydrophoberen Polypropylenoxidteile betroffen sind. Durch diese Hydrophobisierung findet eine Assoziation der lipophilen Propylenoxid-Einheiten zu Mizellen und bei weiterer Temperaturerhöhung und ausreichender Poloxamer-Konzentration die Ausbildung eines Gelgerüstes aus dicht gepackten Mizellen statt. Diese Tensidgele sind optisch isotrop und daher glasklar.

Ein bevorzugtes Poloxamer zur Herstellung der Trägerzusammensetzung der vorliegenden Erfindung ist das Poloxamer 407, welches auch unter der Bezeichnung Kolliphor P 407 vertrieben wird. Poloxamer 407 findet insbesondere bei pharmazeutischen Präparaten und Medizinprodukten Anwendung und weist die folgende Strukturformel auf: 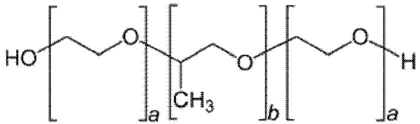 wobei die Blocklängen bei etwa a=101 und b=56 liegen. Während die Verwendung Poloxamer 407 als Ausgangssubstanz zur Herstellung der erfindungsgemäßen Trägerzusammensetzungen besonders bevorzugt wird, können auch andere Poloxamere, wie z.B. Poloxamer 188, verwendet werden.

Wässrige Lösungen von Poloxamer 407 zeigen bei Konzentrationen von 20-30% eine sogenannte Thermogelierung. Dieser Gelierungsprozess ist bei anschließender Temperaturabsenkung vollständig reversibel (Mortensen & Pedersen (1993), Macromolecules 26(4), S. 805-812). Der Thermogelierpunkt (TGP) einer Zusammensetzung, d.h. die Temperatur, bei der eine solche Sol-Gel-Umwandlung stattfindet, kann mittels Oszillationsrheologie problemlos bestimmt werden.

Vorzugsweise liegt der Anteil der Ethylenoxid(EO)-Propylenoxid(PO)-Blockcopolymere in der Trägerzusammensetzung vorzugsweise zwischen etwa 10% und etwa 40% (w/w), und vorzugsweise zwischen etwa 20% und etwa 37% (w/w). So kann der Anteil der Ethylenoxid(EO)-Propylenoxid(PO)-Blockcopolymere in der Trägerzusammensetzung z.B. 10%, 15%, 20%, 25%, 30%, 35%, oder 40% betragen. Der Anteil des Wassers in den Trägerzusammensetzungen liegt üblicherweise zwischen etwa 60% und etwa 90% (w/w). So kann der Anteil des Wassers in den Trägerzusammensetzungen etwa 60%, 65%, 70%, 75%, 80%, 85%, oder 90% (w/w) betragen.

In einer bevorzugten Ausführungsform weisen die Ethylenoxid(EO)-Propylenoxid(PO)-Blockcopolymere in der Trägerzusammensetzung eine Verteilung der Molekularmasse zwischen etwa 1.000 g/mol und 70.000 g/mol auf. In einer besonders bevorzugten Ausführungsform bestehen die Ethylenoxid(EO)-Propylenoxid(PO)-Blockcopolymere in der Trägerzusammensetzung zu mindestens 30% (w/w), vorzugsweise 40% (w/w) aus einem Poloxamer, vorzugsweise aus Poloxamer 407, das eine mittlere Molekularmasse im Bereich von 9.800 bis 14.600 g/mol aufweist.

Im Rahmen der Erfindung wurde überraschend herausgefunden, dass die Viskosität eines Hydrogels auf Basis eines Poloxamers, wie z.B. Poloxamer 407, durch Zugabe von Silica-Nanopartikeln erheblich erhöht werden kann. Wie in den nachstehenden Beispielen gezeigt wird erhöht der Zusatz der Nanaopartikel die Viskosität von Hydrogelen auf Poloxamer-Basis um das 10-fache und ermöglicht damit die Verwendung der Hydrogele als formbare pastöse Trägermaterialien. Der Anteil der Silica-Nanopartikel in der Trägerzusammensetzung der Erfindung liegt vorzugsweise zwischen etwa 2% und etwa 12% (w/w), vorzugsweise im Bereich zwischen etwa 3,5% und etwa 5% (w/w). Es ist besonders bevorzugt, dass der Anteil der Silica-Nanopartikel in der Trägerzusammensetzung bei etwa 2%, 3%, 4%, 5%, 6%, 7%, 8%, 9%, 10%, 11% oder 12% (w/w) liegt.

Die Silica-Nanopartikel weisen eine Größe zwischen etwa 0,5 nm und etwa 10 nm, bevorzugt zwischen etwa 0,5 nm und etwa 1,5 nm. Bevorzugt sollen die Silica-Nanopartikel keine fraktalen Cluster bilden. Sofern die Silica-Nanopartikel fraktale Aggregationscluster bilden, weisen diese Cluster vorzugsweise eine Größe von weniger als etwa 500 nm, stärker bevorzugt weniger als etwa 200 nm, und noch stärker bevorzugt weniger als etwa 100 nm auf. Vorzugsweise werden Aggregationscluster verwendet, die weniger als 15 Nanopartikel umfassen, z.B. weniger als 10 oder weniger als 5.

Da ein auf Wasser basierendes Gel die Grundlage für die erfindungsgemäße Trägerzusammensetzung ist, bietet es sich an, die Silica-Nanopartikel aus einer Natriumwasserglaslösung herzustellen. Bei der Verwendung einer typischen Natriumwasserglaslösung als Ausgangssubstrat mit einer SiO₂ Konzentrationen von etwa 27% und einer Na₂O-Konzentration von etwa 8% entstehen Sol-Teilchen von etwa 0.5 nm. Die Natriumionen können durch einen Ionenaustauscher gegen Wasserstoff ersetzt werden, sodass ein reines Silica-Sol entsteht. Da die Teilchenoberfläche der Silica-Nanopartikel mit den Polymer-Molekülen wechselwirkt, sollen bevorzugt nicht aggregierte Sol-Teilchen für die Trägerzusammensetzung verwendet werden. Nach dem Ionenaustausch liegt der pH-Wert des Sols in der Regel 2 bis 3. Ein Aggregieren der Solteilchen zu Clustern erfolgt bei diesem pH-Wert sehr langsam und kann durch Kühlen des Sol weiter verlangsamt werden. Eine typische SiO₂-Konzentration, bei der problemlos eine Weiterverarbeitung erfolgen kann, ist 6%. Durch Kühlen und schnelle Verarbeitung sind SiO₂-Konzentrationen von bis zu 12% möglich. Ein Stabilisieren des Sols ist auch durch Änderung des pH-Werts auf einen Wert größer als 7 möglich. Da letztendlich ein implantierbares Biomaterial hergestellt werden soll, das einen pH-Wert von etwa 7,5 haben sollte, ist ein pH-Wert über 8 nicht bevorzugt. Nach Herstellen des Sols kann dieses unmittelbar mit einer Lösung des Polymers vermischt werden. Alternativ kann das Polymer auch direkt in das Sol eingerührt werden.

Die erfindungsgemäßen Trägerzusammensetzungen sind ausreichend viskos um eine gute Formbarkeit und eine hohe Klebrigkeit zu gewährleisten. Vorzugsweise weisen die vorliegend bereitgestellten Trägerzusammensetzungen eine Viskosität im Bereich von mehr als 900 Pas, vorzugsweise mehr als 1.000 Pas, bei Messung der Viskosität in Abhängigkeit von der Scherrate unter Verwendung des StrainSweep Test, Oszillationsrheometer ARES - T.A. Instruments, Scherrate 50 1/s.

Besonders bevorzugte Trägerzusammensetzungen weisen die folgende Zusammensetzung auf:
- Anteil EO-PO-Blockcopolymere zwischen etwa 10% und etwa 40% (w/w), Anteil Silica-Nanopartikel zwischen etwa 2% und etwa 12% (w/w);
- Anteil EO-PO-Blockcopolymere zwischen etwa 15% und etwa 37% (w/w), Anteil Silica-Nanopartikel zwischen etwa 3% und etwa 10% (w/w);
- Anteil EO-PO-Blockcopolymere zwischen etwa 20% und etwa 30% (w/w), Anteil Silica-Nanopartikel zwischen etwa 3% und etwa 6% (w/w);
- Anteil EO-PO-Blockcopolymere zwischen etwa 20% und etwa 25% (w/w), Anteil Silica-Nanopartikel zwischen etwa 3% und etwa 5% (w/w).

Ein weiterer Aspekt betrifft ein Verfahren zur Herstellung einer Trägerzusammensetzung für partikuläre und granuläre Knochenersatzmaterialien, bei dem man:
(a) eine wässrige Lösung eines Ethylenoxid(EO)-Propylenoxid(PO)-Blockcopolymers oder eines Gemisches aus Ethylenoxid(EO)-Propylenoxid(PO)-Blockcopolymeren, und
(b) Silica-Nanopartikel mit einer Größe zwischen etwa 0,5 nm und etwa 10 nm.
miteinander vermischt und zu einem Hydrogel formuliert. Vorzugsweise liegen beide Komponenten zum Zeitpunkt des Mischens als wässrige Lösungen vor.

Die oben beschriebenen Trägerzusammensetzungen lassen sich für die Herstellung von Knochenersatzmaterialien verwenden, indem herkömmliche osteokonduktive oder osteoinduktive Partikel oder Granulate mit den Trägerzusammensetzungen kombiniert werden. In einem weiteren Aspekt stellt die Erfindung somit ein Knochenersatzmaterial bereit, das mindestens die folgenden Komponenten umfassend:
(a) eine wie oben beschriebene Trägerzusammensetzung; und
(b) osteokonduktive und/oder osteoinduktive Partikel oder ein osteokonduktives und/oder osteoinduktives Granulat.

Dabei können grundsätzlich alle bekannten osteokonduktiven und/oder osteoinduktiven Partikel und Granulate mit den neuartigen Trägerzusammensetzungen verwendet werden. Als "osteoinduktiv" werden vorliegend Partikel und Granulate bezeichnet, die in der Lage sind, nach der Implantation die Knochenneubildung anzuregen, wobei auch eine ektope Knochenbildung auftritt (Knochenbildung im Muskel oder im Fettgewebe). Als "osteokonduktiv" werden hingegen vorliegend Partikel und Granulate bezeichnet, die in der Lage sind, nach der Implantation als Gerüststruktur für die Knochenneubildung zu dienen.

Die neuartigen Trägerzusammensetzungen eignen sich zur Verwendung mit allen bekannten alloplastischen, xenogenen und allogenen Materialien. So können die Trägerzusammensetzungen insbesondere mit synthetischen keramischen Granulaten verwendet werden, wie z.B. Tricalciumphosphat (TCP)-Keramiken oder Hydroxyapatit (HA)-Keramiken. Bei der Herstellung von synthetischen Keramiken werden pulverförmige Ausgangsstoffe bei hohem Druck und Temperaturen von 1.000 bis 1.500°C einem Sinterungsprozess unterzogen. Das bevorzugte Calcium-Phosphor-Verhältnis der Keramiken liegt zwischen 1,5 und 1,7. Vorzugsweise sind die Keramiken porös, sodass eine ausreichende Osteointegration mittels Durchdringung der Keramik mit neuem Knochengewebe gewährleistet ist. Poren einer Größe von 150-500 µm sind für das Einwachsen von Knochen und für die Resorption optimal. Kleinere Porengrößen führen in der Regel lediglich zum Anwachsen von neuem Knochengewebe.

Neben Keramiken können auch Biogläser Verwendung finden. Biogläser, wie z.B. Biogran^{®}, sind amorphe Materialien, die saure Oxide, wie Phosphorpentoxid, Siliciumdioxid oder Aluminiumoxid, und basische Oxide, wie Calciumoxid, Magnesiumoxid und Zinkoxid umfassen. Bei der Herstellung werden die Oxide gemischt und in einem mehrstündigen Prozess bei hohen Temperaturen von etwa 1.500°C geschmolzen. Das entstandene Bioglass stellt ein dreidimensionales Phosphoroxid-Siliciumoxid-Netzwerk dar, an das sich die entsprechenden Metallionen der basischen Oxide anlagern. Biogläser sind in kompakter Form und auch in poröser Form erhältlich. Die Bioaktivität der Oberfläche ermöglicht ein Anwachsen von Knochengewebe.

Neben den oben genannten Granulaten, die in der Regel eine Größe im Bereich von 0,1 bis 5 mm aufweisen, können auch Partikel, wie z.B. Mikroteilchen verwendet werden. Vorzugsweise haben diese osteokonduktiven oder osteoinduktiven Partikel eine Größe zwischen etwa 5 µm und 100 µm, vorzugsweise zwischen etwa 20 µm und 40 µm.

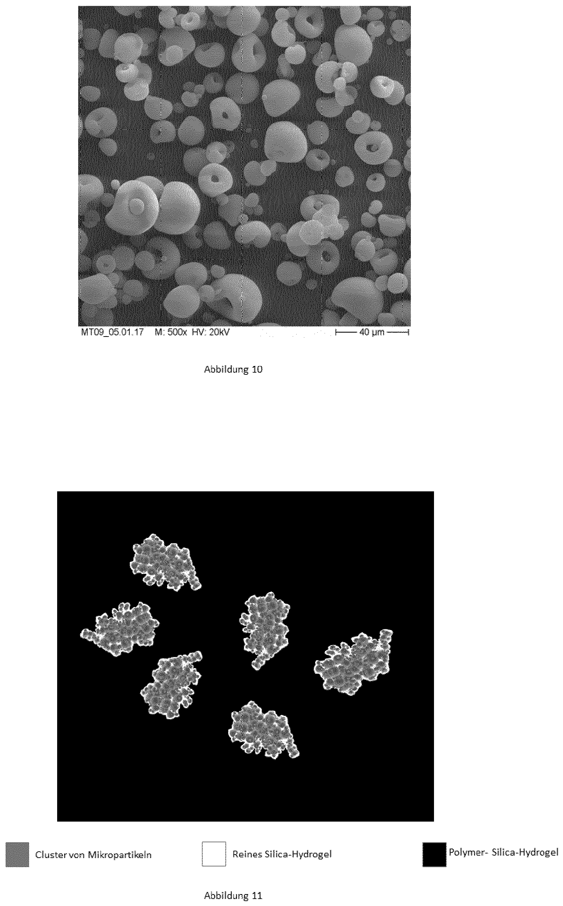

Bei den osteokonduktiven oder osteoinduktiven Partikeln kann es sich z.B. um Hohlkugeln mit einer Öffnung (Donut-Form) handeln. Diese können einen Durchmesser im Bereich von 40 µm aufweisen. Abbildung 10 zeigt beispielhaft osteokonduktive oder osteoinduktive Partikel in Form von Hohlkugeln. Wie im Folgenden erläutert wird sollten derartige Teilchen zur Vermeidung von Lufteinschlüssen vor Einbettung in das Poloxamer-Hydrogel mit einem Silica-Hydrogel ummantelt werden. Das daraus resultierende Knochenersatzmaterial kann in einer durch herkömmliche Kanülen injizierbare Form hergestellt werden. Mikroteilchen, wie z.B. Hohlkugeln, können auch in Clustern verwendet werden. Solche Cluster weisen vorzugsweise eine Größe zwischen etwa 100 µm und 3.000 µm auf. Auch die Cluster sollten von einem Silica-Hydrogel ummantelt sein, bevor eine Einbettung in das Poloxamer-Silica-Hydrogel erfolgt, wie es in Abbildung 11 schematisch dargestellt ist.

In einer besonders bevorzugten Ausführungsform bestehen die osteokonduktiven und/oder osteoinduktiven Partikel oder das osteokonduktive und/oder osteoinduktive Granulat aus Hydroxyapatit-Kristalliten mit einer Morphologie des biologischen Hydroxyapatits des Knochens, die in einer Matrix aus Silica-Xerogel eingebettet sind. Auch diese Partikel oder Granulate können zur Vermeidung von Lufteinschlüssen vor Einbettung in das Poloxamer-Hydrogel mit einem Silica-Hydrogel ummantelt werden.

Vorzugsweise handelt es sich bei den osteokonduktiven oder osteoinduktiven Partikeln oder Granulaten um poröse Materialien. Poröse oder hochporöse Knochenersatzmaterialien mit einer hohen spezifischen Oberfläche haben entscheidende Vorteile bei der Unterstützung der Knochenregeneration, da autologe Proteine in Wechselwirkung mit der Oberfläche des Materials treten.

Sofern die vorliegend beschriebenen Trägerzusammensetzungen in Verbindung mit porösen oder hochporösen Partikeln oder Granulaten verwendet werden, so werden diese vorzugsweise vor der Einbettung in das Poloxamer-Silica-Hydrogel entsprechend behandelt, um Lufteinschlüsse zu vermeiden. Aufgrund der hohen Viskosität kann das Poloxamer-Silica-Hydrogel in manchen Fällen nicht in die Poren der Partikel oder Granulate eindringen. Die daraus resultierenden Lufteinschlüsse können die Funktionalität des Biomaterials beeinträchtigen oder sogar gänzlich verhindern. Des Weiteren könnten die Polymerketten aus dem Poloxamer-Silica-Hydrogel die Oberfläche des Knochenersatzmaterials bedecken und damit eine Wechselwirkung mit autologen Proteinen verhindern. So können die Poren der Partikel oder Granulate mit einem reinen Silica-Gel behandelt werden, sodass alle Poren gefüllt sind und eine Silica-Hydrogel-Schicht die Partikel oder das Granulat ummantelt. Das Silica-Gel, welches für die Ummantelung verwendet wird, kann eine Silica-Konzentration zwischen etwa 3% und etwa 10% aufweisen. Anschließend können die ummantelten Partikel oder das ummantelte Granulat in das Poloxamer-Silica-Hydrogel eingebettet werden. Ein entsprechendes Verfahren ist in Beispiel 2 beschrieben. Somit betrifft die Erfindung in einer bevorzugten Ausführungsform ein Knochenersatzmaterial, das osteokonduktive und/oder osteoinduktive Partikel, wie z.B. Mikroteilchen, enthält, welche mit einem Silica-Gel ummantelt sind.

Schließlich stellt die vorliegende Erfindung auch ein Verfahren zur Herstellung eines Knochenersatzmaterials bereit, bei dem man
(a) eine wie oben beschriebene Trägerzusammensetzung bereitstellt;
(b) die Trägerzusammensetzung optional mit Gammastrahlung behandelt; und
(c) die Trägerzusammensetzung mit osteokonduktiven und/oder osteoinduktiven Partikeln oder mit einem osteokonduktiven und/oder osteoinduktiven Granulat vermischt.

Bei der Herstellung eines Knochenersatzmaterials auf Basis der neuartigen Trägerzusammensetzung wird zunächst eine wie oben beschriebene Trägerzusammensetzung bereitgestellt. Diese kann vor dem Mischen mit den entsprechenden osteokonduktiven oder osteoinduktiven Partikeln oder Granulaten mit Gammastrahlung behandelt werden, um die Zusammensetzung zu sterilisieren. Die Intensität der Strahlung wird in der Regel zwischen 10 und 50 kGray betragen, vorzugsweise zwischen 17.5 und 30 kGray. Anschließend wird die Trägerzusammensetzung mit osteokonduktiven und/oder osteoinduktiven Partikeln oder mit einem osteokonduktiven und/oder osteoinduktiven Granulat vermischt.

Das Mischen kann im Verhältnis (w/w) von Trägerzusammensetzung zu Partikeln oder Granulat von etwa 5:1, 4:1, 3:1, 2:1, 1:1, 1:2, 1:3, 1:4 oder 1:5 erfolgen. Ein Verhältnis von etwa 1:1 ist bevorzugt. Anschließend kann das so hergestellte Knochenersatzmaterial bis zu seiner weiteren Verwendung gelagert werden. Das Knochenersatzmaterial kann z.B. in einen Applikator gefüllt werden, der die Verabreichung des Materials in eine Defektstelle erleichtert.

In einer bevorzugten Ausführungsform werden die osteokonduktiven und/oder osteoinduktiven Partikel oder das osteokonduktive und/oder osteoinduktive Granulat vor Mischen mit der Trägerzusammensetzung behandelt, um Lufteinschlüsse zu vermeiden. Besonders bevorzugt ist es im Rahmen der Erfindung, dass die osteokonduktiven und/oder osteoinduktiven Partikel oder das osteokonduktive und/oder osteoinduktive Granulat vor dem Vermischen mit der Trägerzusammensetzung wie oben beschrieben einem Silica-Hydrogel ummantelt werden.

Ein weiterer Aspekt betrifft die Verwendung einer wie oben beschriebenen Trägerzusammensetzung zur Herstellung eines Knochenersatzmaterials. Somit betrifft der Aspekt die Verwendung eines Hydrogels, das folgendes umfasst:
(a) ein Ethylenoxid(EO)-Propylenoxid(PO)-Blockcopolymer oder ein Gemisch aus Ethylenoxid(EO)-Propylenoxid(PO)-Blockcopolymeren; und
(b) Silica-Nanopartikel,
zur Herstellung eines Knochenersatzmaterials. Die Herstellung umfasst das Mischen mit osteokonduktiven und/oder osteoinduktiven Partikeln oder mit einem wie oben definierten osteokonduktiven und/oder osteoinduktiven Granulat.

Ein weiterer Aspekt betrifft ein wie oben beschriebenes Knochenersatzmaterial, das mindestens die folgenden Komponenten umfassend:
(a) eine wie oben beschriebene Trägerzusammensetzung; und
(b) wie oben beschriebene osteokonduktive und/oder osteoinduktive Partikel oder ein osteokonduktives und/oder osteoinduktives Granulat,
zur Verwendung in einem Verfahren zur Behandlung von Knochendefekten. Bei den Knochendefekten kann es sich insbesondere um Frakturen, spongiöse Knochendefekte oder Kavitäten handeln.

### BESCHREIBUNG DER FIGUREN

Figur 1 zeigt die Molekülmassenverteilung eines SiO₂-haltigen Hydrogels auf Basis von Kolliphor P 407.
Figur 2 zeigt die Molekülmassenverteilung eines SiO₂-haltigen Hydrogels auf Basis von Kolliphor P 407 nach Gamma-Bestrahlung.
Figur 3 zeigt die Molekülmassenverteilung eines SiO₂-haltigen Hydrogels auf Basis von Kolliphor P 407 nach Lagerung des Hydrogels für 55 Tage bei 60°C.
Figur 4a zeigt die Ergebnisse der Messung der Viskosität in Abhängigkeit von der Scherrate. Figur 4b zeigt die Ergebnisse der Messung des Schermoduls in Abhängigkeit von der Frequenz.
Figur 5 zeigt die Entstehung von Lamellarstrukturen unter dem Polarisationsmikroskop.
Figur 6 zeigt die Anwendung des erfindungsgemäßen Knochenersatzmaterials mit einem Applikator.
Figur 7 zeigt das Ergebnis einer Untersuchung des erfindungsgemäßen Knochenersatzmaterials nach beschleunigter Alterung mittels Auflichtmikroskopie.
Figur 8 zeigt das Ergebnis einer HE-Färbung 4 Wochen nach Implantation des erfindungsgemäßen Knochenersatzmaterials in den Hinterlauf eines Kaninchens.
Figur 9 zeigt das Ergebnis einer der histomorphometrischen Auswertung nach Implantation des erfindungsgemäßen Knochenersatzmaterials in den Hinterlauf eines Kaninchens.
Figur 10 zeigt die Verwendung von Mikroteilchen in Form von Hohlkugeln mit einer Öffnung und einem Durchmesser von etwa 40 µm in dem erfindungsgemäßen Knochenersatzmaterial.
Figur 11 zeigt schematisch die Ummantelung von Clustern aus Mikroteilchen mit reinem Silica-Hydrogel vor der Einbettung in das Poloxamer-Silica-Hydrogel.
Figur 12a zeigt die Auftragung der Scherung ["shear stress") in Abhängigkeit von der Scherrate ("shear rate") für unterschiedliche Zusammensetzungen des Trägermaterials: A 19,6% Kolliphor P 407, 0% SiO₂; B 19,6% Kolliphor P 407, 4,8% SiO₂; C 36,0% Kolliphor P 407, 0% SiO₂; D 36,0% Kolliphor P 407, 3,8% SiO₂.
Figur 12b zeigt den komplexen Schubmodul (Speichermodul ["storage modulus] G'; Verlustmodul ["loss modulus"] G") für unterschiedliche Trägermaterialzusammensetzungen: A 36,0% Kolliphor P 407, 0% SiO₂; B 36,0% Kolliphor P 407, 5,0% SiO₂;
Figur 12c zeigt den komplexen Schubmodul (Speichermodul G'; Verlustmodul G") für unterschiedliche Trägermaterialzusammensetzungen: A 19,4% Kolliphor P 407, 0% SiO₂; B 19,4% Kolliphor P 407, 4,8% SiO₂.
Figur 12d zeigt den komplexen Schubmodul (Speichermodul G'; Verlustmodul G") für unterschiedliche Trägermaterialzusammensetzungen: A 16,4% Kolliphor P 407, 0% SiO₂; B 16,4% Kolliphor P 407, 5,0% SiO₂; C 16,4% Kolliphor P 407, 7,4% SiO₂.

### BEISPIELE

Die folgenden Beispiele verdeutlichen die Wirksamkeit sowie die Vorteile der erfindungsgemäßen Trägerzusammensetzung und des daraus formulierten Knochenersatzmaterials.

### Beispiel 1: Herstellung von Hydrogelen mit und ohne SiO₂

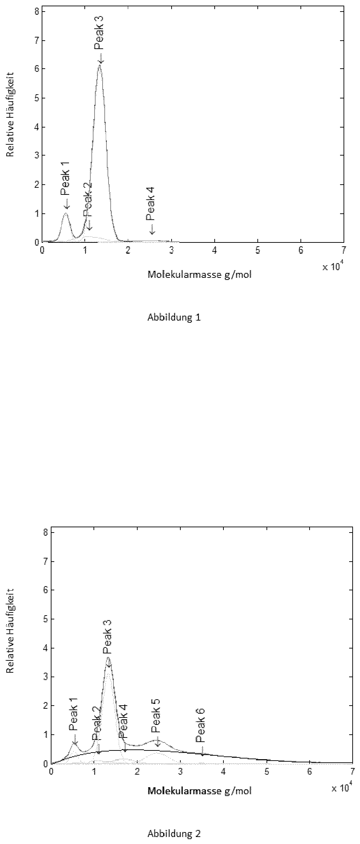

Zu Vergleichszwecken wurden SiO₂-freie und SiO₂-haltige Hydrogele auf Basis von Kolliphor P 407 hergestellt. Für die Herstellung der SiO₂-freien Hydrogele wurden jeweils 23,5 g Kolliphor P 407 von BASF mit 76,5 g Wasser vermischt. Für die SiO₂-haltigen Hydrogele wurde ein Sol durch Ionenaustausch mit einer SiO₂-Konzentration von 4% bzw. 6% hergestellt. Dazu wurde konzentrierte Natriumwasserglaslösung von Merk (Spezifikation: Na₂O: 7,5-8,5%; SiO₂: 25, 5-28, 5%) genutzt und mit Reinstwasser entsprechend verdünnt. Als Ionenaustauscher wurde eine Lewatit MonoPlus SP 112Na+ Säule verwendet. Die Sole haben einen pH Wert von 2,7. Sie wurden auf 5°C herunter gekühlt. In jeweils 76,5 g des Sols wurden 23,5 g Kolliphor P 407 eingerührt. Die so entstandenen Hydrogele enthalten Polymere mit der in Figur 1 dargestellten Molekülmassenverteilung (Molekülmassenverteilung A). Die Molekularmassenverteilung kann mit Chromatographie bestimmt werden. Die Analyse führte zu den folgenden Peaks:

| | | | | | |
|---|---|---|---|---|---|
| Peak 1: | Position: | 5.550 | g/mol, | Anteil: | 17,8 % |
| Peak 2: | Position: | 11.000 | g/mol, | Anteil: | 8,2 % |
| Peak 3: | Position: | 13.470 | g/mol, | Anteil: | 73,1 % |
| Peak 4: | Position: | 25.500 | g/mol, | Anteil: | 0,8 % |

Die Peaks bei 5550 g/mol und 11000g/mol stellen Fragmente von Kolliphor 407 dar. Der Peak bei 25500g/mol entsteht durch Vernetzung zweiter Ketten.

Ein Teil der hergestellten Proben wurde mit Gammastrahlung behandelt (17.5 bis 30 kGray Strahlungsquelle: Kobalt 60, maximale Aktivität 111 PBq). Durch die Gammabestrahlung kommt es zu einer Quervernetzung der Polymerketten. Gleichzeitig werden Ketten auch zerbrochen. Als Ergebnis wird ein Polymer mit einer breiten Verteilung der Molekularmasse erhalten.

Diese Hydrogele enthalten Polymere mit der in Figur 2 dargestellten Molekülmassenverteilung (Molekülmassenverteilung B). Die Analyse führte zu den folgenden Peaks:

| | | | | | |
|---|---|---|---|---|---|
| Peak 1: | Position: | 5.400 | g/mol, | Anteil: | 8,1 % |
| Peak 2: | Position: | 11.000 | g/mol, | Anteil: | 4,0 % |
| Peak 3: | Position: | 13.400 | g/mol, | Anteil: | 37,0 % |
| Peak 4: | Position: | 17.000 | g/mol, | Anteil: | 2,7 % |
| Peak 5: | Position: | 25.500 | g/mol, | Anteil: | 4,2 % |
| Peak 6: | Position: | 35.000 | g/mol, | Anteil: | 0,2 % |

Nach der Bestrahlung betrug der auf die kontinuierliche Verteilung der Massen entfallende Anteil 43.8%. Das ursprüngliche Kolliphor 407 hat nur noch einen Anteil von 37%. Den größten Anteil von 43,8% haben Moleküle mit einer kontinuierlichen Größenverteilung, die bis ca. 70.000 g/mol reicht.

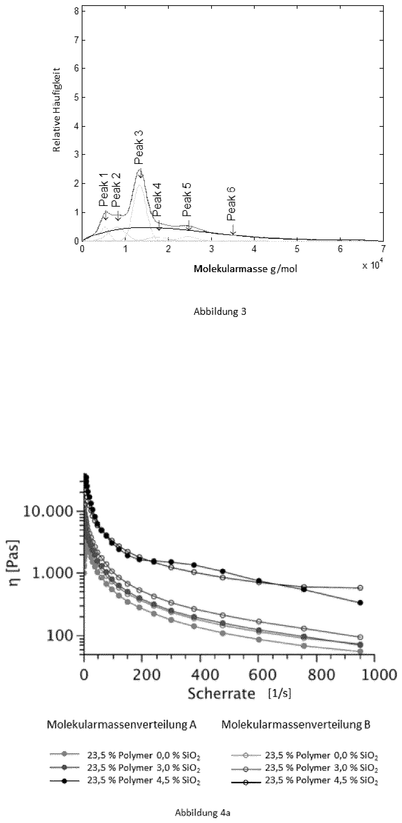

Ein anderer Teil der hergestellten Proben wurde bei erhöhter Temperatur über einen längeren Zeitraum gelagert. Nach Lagerung für 55 Tage bei 60°C enthielten die Hydrogele Polymere mit der in Figur 3 dargestellten Molekülmassenverteilung (Molekülmassenverteilung C). Die Analyse führte zu den folgenden Peaks:

| | | | | | |
|---|---|---|---|---|---|
| Peak 1: | Position: | 5.350 | g/mol, | Anteil: | 10,6 % |
| Peak 2: | Position: | 8.200 | g/mol, | Anteil: | 13,7 % |
| Peak 3: | Position: | 13.470 | g/mol, | Anteil: | 23,6 % |
| Peak 4: | Position: | 17.700 | g/mol, | Anteil: | 1,9733 % |
| Peak 5: | Position: | 24.700 | g/mol, | Anteil: | 1,5018 % |
| Peak 6: | Position: | 35.000 | g/mol, | Anteil: | 0,0 % |

Es ist zu erkennen, dass auch in diesem Fall Moleküle mit einer Größenverteilung, die von etwa 1.000 g/mol bis etwa 70.000 g/mol reicht, den größten Anteil von 48,7 % zeigen.

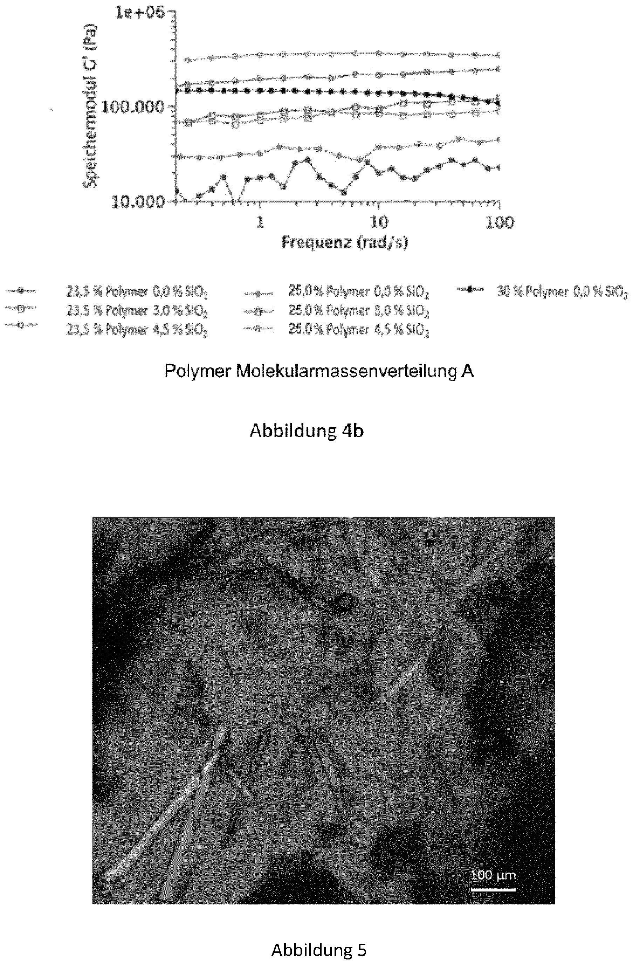

Für alle Proben wurde die Viskosität in Abhängigkeit von der Scherrate gemessen (StrainSweep Test, Oszillationsrheometer ARES - T.A. Instruments). Die Ergebnisse sind in Figur 4a dargestellt. Es ist zu erkennen, dass sich sowohl bei den SiO₂-freien Hydrogelen als auch bei den SiO₂-haltigen Hydrogelen die Viskosität mit der Verbreiterung der Molekülmassenverteilung erhöht. Die Proben mit der Molekülmassenverteilung A sind optisch nicht aktiv, sie zeigen keinen Kontrast im Polarisationsmikroskop. Das bedeutet, dass die Polymere Mizellen bilden. Die Proben mit der Molekülmassenverteilung B sind hingegen optisch aktiv. Sie zeigen einen Kontrast im Polarisationsmikroskop. Dies zeigt, dass die Proben neben Mizellen auch sogenannte Lamellarstrukturen enthalten. Manche Tenside bilden bei hohen Konzentrationen Lamellarstrukturen, in denen sich das Wasser in den polaren Zwischenschichten der Assoziate befindet. Diese optische Anisotropie verändert die Schwingungsebene des linear polarisierten Lichts, sodass unter dem Polarisationsmikroskop charakteristische Hell-Dunkel-Erscheinungen zu erkennen sind. Figur 5 zeigt ein typisches Beispiel, das die Entstehung von Lamellarstrukturen dokumentiert. Ferner ist in Figur 4a zu erkennen, dass die Viskosität mit steigendem SiO₂-Gehalt im Hydrogel stark ansteigt. Bei einer Scherrate von 50 1/s steigt die Viskosität bei Zugabe von 4,5% SiO₂ auf das 10-fache. Dies ist für die Anwendbarkeit der Gele als Träger für Knochenersatzmaterialien von entscheidender Bedeutung.

Darüber hinaus wurde der Schubmodul in Abhängigkeit von der Frequenz gemessen. Diese Messung gibt Aufschluss über das Schwingungsverhalten viskoelastischer Stoffe unter oszillierender Scherbelastung und lässt Rückschlüsse über die Wechselwirkung der Moleküle im System zu. In Abbildung 4b ist der Speicheranteil des Schermoduls in Abhängigkeit von der Frequenz für unterschiedliche Hydrogele dargestellt. Gewählt wurde hier ein Polymer mit der Molekülmassenverteilung A. Zum Einen ist der Effekt zu erkennen, dass der Speicheranteil des Schermoduls mit steigender Polymer-Konzentration größer wurde. Zum anderen stieg der Speicheranteil des Schermoduls mit steigendem SiO₂ stark an. Für das Beispiel mit 25% Polymer-Anteil vergrößerte sich der Speicheranteil um das 10-fache, wenn 4,5% Silica-Nanopartikel im Gel vorhanden waren. Dies zeigt die für die Anwendung der Gele wichtige Wechselwirkung zwischen den Polymerketten und den Silica-Nanopartikeln.

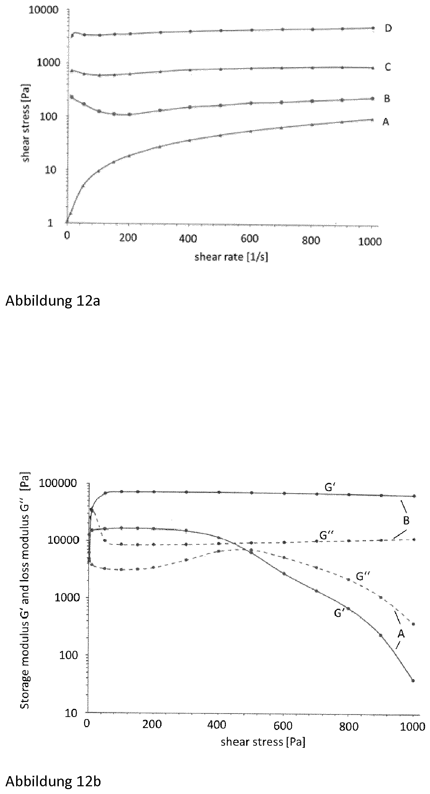

In Abbildung 12a ist die Scherung in Abhängigkeit von der Scherrate für unterschiedliche Zusammensetzungen des Trägermaterials dargestellt. Die Messungen der Scherung erfolgten bei 20°C. Kurve A entspricht dem Trägermaterial mit 19,6% Kolliphor ohne Silica-Nanopartikel. Der Kurvenverlauf entspricht dem einer Flüssigkeit, da bei diesem Kolliphor-Anteil die Gelbildung erst bei etwa 25°C beginnt. Die Kurven B, C und D zeigen einen typischen Verlauf für Hydrogele. Es ist eine Fließgrenze erkennbar (Scherung, bei der das Material beginnt zu fließen). Kurve B zeigt, dass die Zugabe von 4,8% SiO₂ die Flüssigkeit zu einem Gel umwandelt. Kurve C entspricht dem Trägermaterial mit 36,0% Kolliphor ohne Silica-Nanopartikel. Durch Mizellen-Bildung entsteht hier bei 20°C ein Gel. Wird zu dieser Probe 3,8% SiO₂ gegeben, ist eine viel höhere Scherung notwendig, um das Material zum Fließen zu bringen. Die Gelbildung basiert hier auf Wechselwirkung der Polymere mit den Silica-Nanopartikeln.

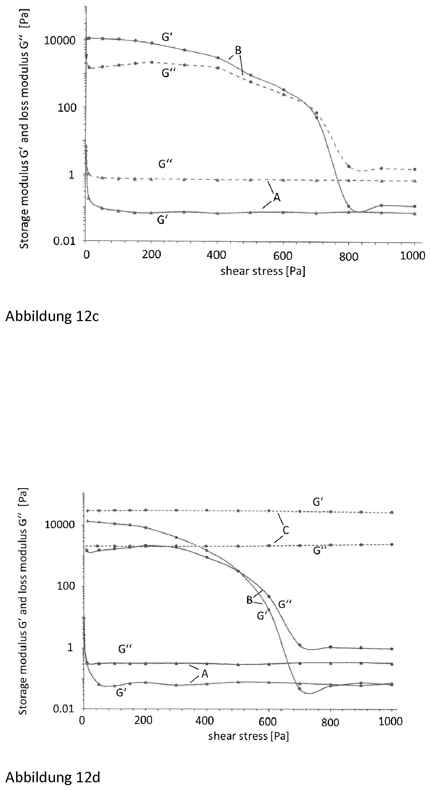

Diesen Effekt dokumentieren auch die Messungen des komplexen Schermoduls in Abhängigkeit von der Scherung, die in Abbildungen 12b, 12c und 12d dargestellt sind. Die Messungen wurden bei 20°C durchgeführt. Ist im Kurvenverlauf der Speicheranteil G' größer als der Verlustanteil G", so handelt es sich bei dem Material um ein Gel. Schneiden sich die beiden Kurven, so beginnt das Material zu fließen. Ist im Kurvenverlauf der Verlustanteil G" größer als der Speicheranteil G', so deutet das Verhalten auf eine Flüssigkeit.

Figur 12b zeigt das Verhalten eines Trägermaterials mit 36,0% Kolliphor. Ohne Silica-Nanopartikel (A) bildet das Material bei 20°C ein Gel, das bei einer Scherung von ca. 500 Pa den Übergang zur Flüssigkeit zeigt. Wird dem Trägermaterial 5% SiO₂ zugefügt (B), so bleibt das Material im gesamten Messbereich ein Gel. Die Kurven von G' und G" nähern sich dabei kaum an. Für die Anwendung bedeutet dies, dass das Trägermaterial mit Silica-Nanopartikeln wesentlich stabiler ist und eine verbesserte Handhabung gewährleistet.

In Abbildung 12c ist dieser Effekt für kleinere Kolliphor-Konzentration dargestellt (19,6%). Ohne Silica-Nanopartikel erfolgt bei 20°C keine Gelbildung. Durch die Zugabe von Silica-Nanopartikeln hingegen kommt es zur Gelbildung. In Abbildung 12c ist die Abhängigkeit dieses Effektes von der SiO₂ Konzentration dokumentiert. Ausgangspunkt ist ein Trägermaterial mit 16,4% Kolliphor, das bei 20°C kein Gel bildet (A). Durch Zugabe von 5,0% SiO₂ wird aus dem Material ein Gel, das bei einer Scherung von ca. 500 Pa den Übergang zur Flüssigkeit zeigt (B). Mit 7% SiO₂ entsteht ein Gel, das sich im gesamten Messbereich als stabil erweist (C). Diese Ergebnisse zeigen, dass durch Veränderung des Verhältnisses von Kolliphor, Silica-Nanopartikeln und Wasser die rheologischen Eigenschaften der Zusammensetzung moduliert werden können. Dadurch ist es möglich, das Trägermaterial für verschiedene Anwendungen gezielt zu optimieren.

### Beispiel 2: Einbettung von porösem Knochenersatzmaterial

Es wurden osteoinduktive Granulatkörner aus Hydroxyapatit (HA) verwendet, die eine Tannenzapfen-Form aufweisen (Nanobone, Artoss GmbH, Rostock, Deutschland). Diese waren im Mittel 3 mm lang und wiesen einen Durchmesser zwischen 0,5 und 1,0 mm auf. Das HA zeigte eine kristallographische Morphologie, die der von biologischem HA glich. Dieses HA war in eine hochporöse Matrix aus Silica-Xerogel eingebettet. Die Porosität der Granulatkörner betrug ca. 50%, die spezifische Oberfläche etwa 200 m²/g und die Porengrößenverteilung zeigte ein Maximum bei 4 nm.

Die Granulatkörner wurden in einem Massenverhältnis von 1:1 mit einem reinen Silica-Sol mit einer SiO₂ Konzentration von 6% und einem pH Wert von 7.0 getränkt. Im Kontakt mit dem Festkörper geliert das Silica-Sol. Es entstehen Granulatkörner, die mit einem Silica-Gel gefüllt und von diesem ummantelt sind.

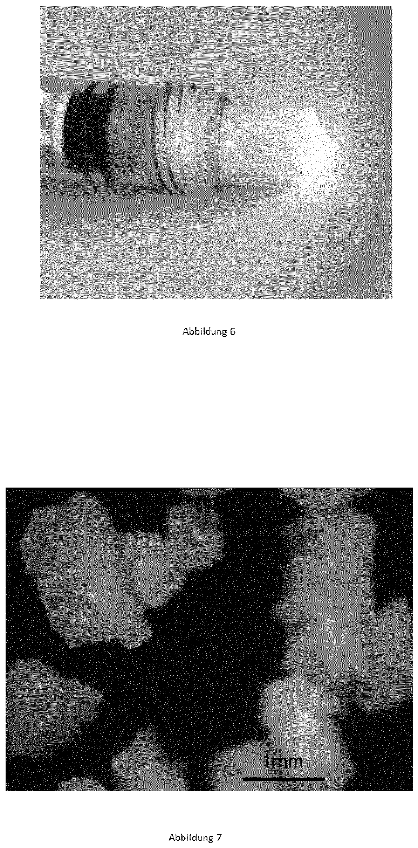

Zur Herstellung des Poloxamer-Silica-Hydrogels wurden 35 g Kolliphor P 407 (BASF) in 65 g Silica-Sol mit 6% SiO₂ Gehalt gerührt. Das Sol wurde zuvor auf 1°C gekühlt. Eine Quervernetzung erfolgt durch eine Gammabestrahlung im Bereich von 17,5 bis 30 kGrey. Dieses Polymer-Silica-Hydrogel wurde mit den ummantelten Granulatkörner in Massenverhältnis 1:1 vermischt. Das entstandene pastöse Knochenersatzmaterial ist sehr gut formbar und kann mit einem Applikator in Knochendefekte eingebracht werden. Figur 6 zeigt die Verwendung des Knochenersatzmaterials mit einem Applikator.

Die Stabilität der Ummantelung der Granulatkörner mit reinem Silica-Hydrogel wurde kontrolliert, indem das Material einer beschleunigten Alterung für 1 Jahr nach ASTM F 1980-07 unterzogen wurde. Nach Entfernen des Poloxamer-Silica-Hydrogels durch Spülen mit Wasser ließen sich im Mikroskop mit reinem Silica-Hydrogel ummantelte Granulatkörner erkennen. Figur 7 zeigt die Analyse der Granulatkörner mittels Auflichtmikroskopie.

### Beispiel 3: Funktionalität im Tierexperiment

Die Versuche wurden an weiblichen Kaninchen (New Zealand White, 3-4 kg, Charles River, Sulzfeld, Deutschland) durchgeführt. Das gemäß Beispiel 2 hergestellte Knochenersatzmaterial wurde bilateral in die Hinterläufe implantiert. Der Schnitt durch die Cutis und Subcutis hat eine Länge von ca. 2,5 cm.

Die Muskulatur wurde ebenfalls in einem kleinen Bereich durchtrennt, um die Verletzungen so gering wie möglich zu halten, und anschließend wurde das Periost vorsichtig an der zu setzenden Defektstelle vom Knochen gelöst. Anschließend wurde jeweils ein zylindrischer Defekt (5 mm im Durchmesser und 10 mm in der Länge) in die lateralen Kondylen der Femora eingebracht. Dazu wurde standardisiert ein Bohrer (Ø 4,5 mm) verwendet. Während der Defektsetzung wurde der Bereich mit 0,9% NaCl-Lösung gespült, um Nekrosen des Knochengewebes aufgrund von Hitzeeinwirkung zu vermeiden.

Die Anästhesie wurde durch Injektion von 10% Ketamin (30-60 mg/kg Körpergewicht) und 2% Xylazin (5 mg/kg Körpergewicht) subkutan in die Nackenfalte eingeleitet. Nach 10 min erfolgte eine Gabe von 0,3 ml Atropin (0,5 mg/ml). Zusätzlich wurden Novaminsulfon (500 mg/ml) als Analgetikum und Penicillin G (intramuskulär 150.000 i.E.) als Antibiotikum injiziert. Eine Lokalanästhesie wurde mit 2 ml Xylocitin-loc (2 %/ml) durchgeführt. Der Wundbereich wurde nach der Implantation mit Gentamicin (80 mg/2 ml, 1:5 Verdünnung mit NaCl) gespült. Der Wundverschluss (Punktnaht) wurde mittels Vicryl-Nahtmaterial vorgenommen.

Nach Versuchszeiträumen von 4, 8 und 12 Wochen wurden die entsprechenden Versuchsgruppen aus dem Versuch genommen. Die Euthanasie erfolgte am narkotisierten Tier (10% Ketamin und 2% Xylazin, subkutan) durch Verwendung von Release^{®} (300mg/ml entsprechend: 1 ml/kg Körpergewicht) intravenös. Für die Auswertung wurden histologische Schnitte angefertigt. Hierzu wurden die Defektregionen explantiert, entkalkt und in Paraffin eingebettet. Es erfolgte eine Hämatoxylin-Eosin-Färbung.

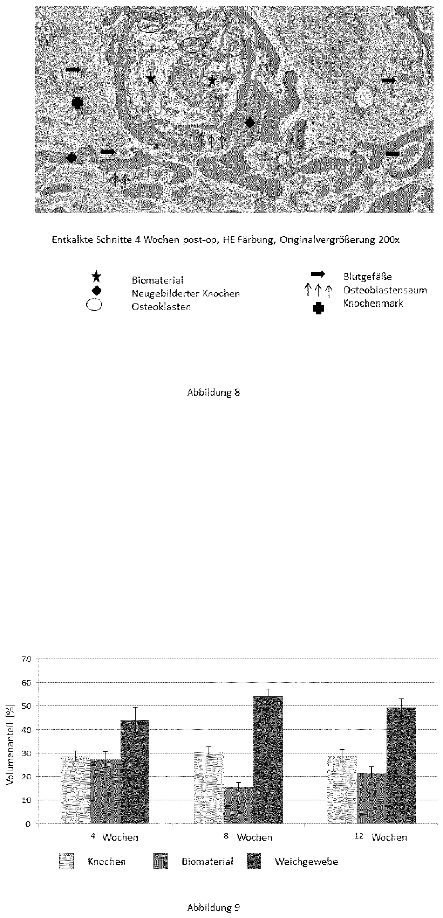

Ergebnis: Nach 4 Wochen war weder das Polymer-Silica-Hydrogel noch das reine Silica-Hydrogel nachweisbar. Es erfolgte eine vollständige Resorption. Änderungen im zeitlichen Ablauf zu im Patientenblut eingebetteten Granulaten waren bei der Defektheilung nicht nachweisbar. Figur 8 zeigt eine histologische Abbildung (HE-Färbung) 4 Wochen nach dem Eingriff. Knochenneubildung und Resorption der Granulatkörner wird durch die ursprüngliche Einbettung in die beiden Hydrogele nicht beeinflusst. Die Ergebnisse der histomorphometrischen Auswertung der Tierexperimente sind in Figur 9 dargestellt. Es ist eine Defektheilung dokumentiert.

## Patentansprüche

1. Trägerzusammensetzung für partikuläre und granuläre Knochenersatzmaterialien, wobei die Trägerzusammensetzung ein Hydrogel ist, das Folgendes umfasst:
(a) ein Ethylenoxid(EO)-Propylenoxid(PO)-Blockcopolymer oder ein Gemisch aus Ethylenoxid(EO)-Propylenoxid(PO)-Blockcopolymeren; und
(b) Silica-Nanopartikel mit einer Größe zwischen etwa 0,5 nm und etwa 10 nm.

2. Trägerzusammensetzung nach Anspruch 1, wobei der Anteil von Wasser im Hydrogel im Bereich von 60% bis 90% liegt.

3. Trägerzusammensetzung nach Anspruch 1 und 2, wobei der Anteil der Ethylenoxid(EO)-Propylenoxid(PO)-Blockcopolymere im Hydrogel zwischen etwa 10% und 40% (w/w) liegt, vorzugsweise zwischen etwa 20% und 37% (w/w).

4. Trägerzusammensetzung nach einem der Ansprüche 1-3, wobei der Anteil der Silica-Nanopartikel zwischen etwa 2% und 12% (w/w) liegt, vorzugsweise im Bereich zwischen 3,5 % und 5% (w/w).

5. Trägerzusammensetzung nach einem der Ansprüche 1-4, wobei die Silica-Nanopartikel eine Größe zwischen 0,5 nm und 1,5 nm.

6. Trägerzusammensetzung nach einem der Ansprüche 1-5, wobei die Silica-Nanopartikel fraktale Aggregationscluster bilden, die eine durchschnittliche Größe von weniger als 200 nm aufweisen.

7. Trägerzusammensetzung nach einem der Ansprüche 1-6, wobei die Ethylenoxid(EO)-Propylenoxid(PO)-Blockcopolymere in der Trägerzusammensetzung eine Verteilung der Molekularmasse zwischen etwa 1.000 g/mol und 70.000 g/mol aufweisen.

8. Trägerzusammensetzung nach einem der Ansprüche 1-7, wobei die Ethylenoxid(EO)-Propylenoxid(PO)-Blockcopolymere in der Trägerzusammensetzung zu mindestens 30% (w/w), vorzugsweise 40% (w/w) aus einem Poloxamer, vorzugsweise aus Poloxamer 407, das eine mittlere Molekularmasse im Bereich von 9.800 bis 14.600 g/mol aufweist.

9. Knochenersatzmaterial, umfassend:
(a) eine Trägerzusammensetzung nach einem der Ansprüche 1-8;
(b) osteokonduktive und/oder osteoinduktive Partikel oder ein osteokonduktives und/oder osteoinduktives Granulat.

10. Knochenersatzmaterial nach Anspruch 9, wobei die osteokonduktiven oder osteoinduktiven Partikel eine Größe zwischen etwa 5 µm und 100 µm aufweisen, vorzugsweise zwischen etwa 20 µm und 40 µm.

11. Knochenersatzmaterial nach Anspruch 9 oder 10, wobei es sich bei den osteokonduktiven oder osteoinduktiven Partikeln um Hohlkugeln mit einer Öffnung handelt.

12. Knochenersatzmaterial nach Anspruch 11, wobei die Hohlkugeln Cluster einer Größe zwischen etwa 100 µm und 3.000 µm bilden.

13. Knochenersatzmaterial nach Anspruch 9 oder 10, wobei die osteokonduktiven oder osteoinduktiven Partikel oder das osteokonduktive und/oder osteoinduktive Granulat aus Hydroxyapatit-Kristalliten bestehen, welche die Morphologie des biologischen Hydroxyapatits des Knochens aufweisen und von einer Matrix aus Silica-Xerogel ummantelt sind.

14. Knochenersatzmaterial nach einem der Ansprüche 9-13, wobei die osteokonduktiven und/oder osteoinduktiven Partikel oder das osteokonduktive und/oder osteoinduktive Granulat mit einem Silica-Gel ummantelt sind, wobei die Silica-Konzentration in dem Silica-Gel vorzugsweise zwischen etwa 3% und 10% liegt.

15. Verfahren zur Herstellung eines Knochenersatzmaterials bereit, bei dem man
(a) eine Trägerzusammensetzung nach den Ansprüchen 1-8 bereitstellt;
(b) optional die Trägerzusammensetzung mit Gammastrahlung behandelt; und
(c) die Trägerzusammensetzung mit osteokonduktiven und/oder osteoinduktiven Partikeln oder mit einem osteokonduktiven und/oder osteoinduktiven Granulat vermischt;
wobei die osteokonduktiven und/oder osteoinduktiven Partikeln oder das osteokonduktive und/oder osteoinduktive Granulat vorzugsweise mit einem Silica-Hydrogel ummantelt sind/ist.

## Claims

1. A carrier composition for particulate and granular bone substitute materials, wherein the carrier composition is a hydrogel comprising:
(a) an ethylene oxide (EO)-propylene oxide (PO) block copolymer or a mixture of ethylene oxide (EO)-propylene oxide (PO) block copolymers; and
(b) silica nanoparticles having a size between about 0.5 nm and about 10 nm.

2. The carrier composition according to claim 1, wherein the proportion of water in the hydrogel ranges from 60% to 90%.

3. The carrier composition according to claims 1 and 2, wherein the proportion of ethylene oxide (EO)-propylene oxide (PO) block copolymers in the hydrogel is between about 10% and 40% (w/w), preferably between about 20% and 37% (w/w).

4. A carrier composition according to any one of claims 1-3, wherein the proportion of silica nanoparticles is between about 2% and 12% (w/w), preferably in the range between 3.5% and 5% (w/w).

5. A carrier composition according to any of claims 1-4, wherein the silica nanoparticles have a size between 0.5 nm and 1.5 nm.

6. A carrier composition according to any one of claims 1-5, wherein the silica nanoparticles form fractal aggregation clusters having an average size of less than 200 nm.

7. A carrier composition according to any of claims 1-6, wherein the ethylene oxide (EO)-propylene oxide (PO) block copolymers in the carrier composition have a molecular weight distribution between about 1,000 g/mol and 70,000 g/mol.

8. A carrier composition according to any one of claims 1-7, wherein at least 30% (w/w), preferably 40% (w/w), of the ethylene oxide (EO)-propylene oxide (PO) block copolymers in the carrier composition consist of a poloxamer, preferably poloxamer 407, having an average molecular weight in the range of 9,800 to 14,600 g/mol.

9. A bone substitute material comprising:
(a) a carrier composition according to any of claims 1-8;
(b) osteoconductive and/or osteoinductive particles or osteoconductive and/or osteoinductive granules.

10. A bone substitute material according to claim 9, wherein the osteoconductive or osteoinductive particles have a size between about 5 µm and 100 µm, preferably between about 20 µm and 40 µm.

11. A bone substitute material according to claim 9 or 10, wherein the osteoconductive or osteoinductive particles are hollow spheres having an opening.

12. A bone substitute material according to claim 11, wherein the hollow spheres form clusters of a size between about 100 µm and 3,000 µm.

13. A bone substitute material according to claim 9 or 10, wherein the osteoconductive or osteoinductive particles or the osteoconductive and/or osteoinductive granules consist of hydroxyapatite crystallites which have the morphology of biological hydroxyapatite of the bone and are coated with a matrix of silica xerogel.

14. A bone substitute material according to any of claims 9-13, wherein the osteoconductive and/or osteoinductive particles or the osteoconductive and/or osteoinductive granules are coated with a silica gel, wherein the silica concentration in the silica gel is preferably between about 3% and 10%.

15. A method for the preparation of a bone substitute material comprising
(a) providing a carrier composition according to claims 1-8;
(b) optionally treating the carrier composition with gamma radiation; and
(c) mixing the carrier composition with osteoconductive and/or osteoinductive particles or osteoconductive and/or osteoinductive granules;
wherein the osteoconductive and/or osteoinductive particles or the osteoconductive and/or osteoinductive granules are preferably coated with a silica hydrogel.

## Revendications

1. Composition porteuse pour des matériaux substituts osseux particulaires et granulaires, dans laquelle la composition porteuse est un hydrogel, qui comprend ce qui suit :
(a) un copolymère séquencé oxyde d'éthylène (EO)-oxyde de propylène (PO) ou un mélange de copolymères séquencés oxyde d'éthylène (EO)-oxyde de propylène (PO) ; et
(b) des nanoparticules de silice d'une taille comprise entre environ 0,5 nm et environ 10 nm.

2. Composition porteuse selon la revendication 1, dans laquelle la proportion d'eau dans l'hydrogel se situe dans la plage de 60 % à 90 %.

3. Composition porteuse selon la revendication 1 et 2, dans laquelle la proportion de copolymères séquencés oxyde d'éthylène (EO)-oxyde de propylène (PO) dans l'hydrogel est comprise entre environ 10 % et 40 % (p/p), de préférence entre environ 20 % et 37 % (p/p).

4. Composition porteuse selon l'une quelconque des revendications 1-3, dans laquelle la proportion de nanoparticules de silice est comprise entre environ 2 % et 12 % (p/p), de préférence dans la plage comprise entre 3,5 % et 5 % (p/p).

5. Composition porteuse selon l'une quelconque des revendications 1-4, dans laquelle les nanoparticules de silice une taille comprise entre 0,5 nm et 1,5 nm.

6. Composition porteuse selon l'une quelconque des revendications 1-5, dans laquelle les nanoparticules de silice forment des agrégats fractals, qui présentent une taille moyenne inférieure à 200 nm.

7. Composition porteuse selon l'une quelconque des revendications 1-6, dans laquelle les copolymères séquencés oxyde d'éthylène (EO)-oxyde de propylène (PO) dans la composition porteuse présentent une distribution de la masse moléculaire comprise entre environ 1 000 g/mol et 70 000 g/mol.

8. Composition porteuse selon l'une quelconque des revendications 1-7, dans laquelle les copolymères séquencés oxyde d'éthylène (EO)-oxyde de propylène (PO) dans la composition porteuse sont constitués à au moins 30 % (p/p), de préférence 40 % (p/p) d'un poloxamère, de préférence de poloxamère 407, qui a une masse moléculaire moyenne dans la plage de 9 800 à 14 600 g/mol.

9. Matériau substitut osseux, comprenant :
(a) une composition porteuse selon l'une quelconque des revendications 1-8 ;
(b) des particules ostéoconductrices et/ou ostéoinductrices ou des granulés ostéoconducteurs et/ou ostéoinducteurs.

10. Matériau substitut osseux selon la revendication 9, dans lequel les particules ostéoconductrices ou ostéoinductrices présentent une taille comprise entre environ 5 µm et 100 µm, de préférence entre environ 20 µm et 40 µm.

11. Matériau substitut osseux selon la revendication 9 ou 10, dans lequel les particules ostéoconductrices ou ostéoinductrices sont des sphères creuses avec une ouverture.

12. Matériau substitut osseux selon la revendication 11, dans lequel les sphères creuses forment des agrégats d'une taille comprise entre environ 100 µm et 3 000 µm.

13. Matériau substitut osseux selon la revendication 9 ou 10, dans lequel les particules ostéoconductrices ou ostéoinductrices ou les granulés ostéoconducteurs et/ou ostéoinducteurs sont constitués de cristallites d'hydroxyapatite, lesquels présentent la morphologie de l'hydroxyapatite biologique de l'os et sont enrobés d'une matrice de xérogel de silice.

14. Matériau substitut osseux selon l'une quelconque des revendications 9-13, dans lequel les particules ostéoconductrices et/ou ostéoinductrices ou les granulés ostéoconducteurs et/ou ostéoinducteurs sont enrobés d'un gel de silice, dans lequel la concentration en silice dans le gel de silice est de préférence comprise entre environ 3 % et 10 %.

15. Procédé pour fabriquer un matériau substitut osseux, selon lequel on
(a) fournit une composition porteuse selon les revendications 1-8 ;
(b) éventuellement, traite la composition porteuse avec un rayonnement gamma ; et
(c) la composition porteuse est mélangée avec des particules ostéoconductrices et/ou ostéoinductrices ou avec des granulés ostéoconducteurs et/ou ostéoinducteurs ;
dans lequel les particules ostéoconductrices et/ou ostéoinductrices ou les granulés ostéoconducteurs et/ou ostéoinducteurs sont de préférence enrobés d'un hydrogel de silice.
